Europäisches Patentamt

European Patent Office

Office européen des brevets

(11) Publication number: **0 158 397**
A2

# EUROPEAN PATENT APPLICATION

(21) Application number: **85200493.6**

(22) Date of filing: **01.04.85**

(51) Int. Cl.⁴: **A 61 M 25/00**

(30) Priority: **12.04.84 NL 8401181**

(43) Date of publication of application:
**16.10.85 Bulletin 85/42**

(84) Designated Contracting States:
**AT BE CH DE FR GB IT LI LU NL SE**

(71) Applicant: **Porcelijn, Tom
Cornelis Schuytstraat 4hs
NL-1071 JH Amsterdam(NL)**

(72) Inventor: **Porcelijn, Tom
Cornelis Schuytstraat 4hs
NL-1071 JH Amsterdam(NL)**

(74) Representative: **Kupecz, Arpad et al,
Octrooibureau Los en Stigter B.V. Postbox 20052
NL-1000 HB Amsterdam(NL)**

(54) Instrument for local anaesthesia and accompanying plug.

(57) Instrument for local anaesthesia comprising a tubular canula (1) and a stylet (2) including a sharply pointed shaft (21) fitting in the canula (1), which consists of an electrically insulating material and in the operative position of the instrument surrounds the shaft (21) of the stylet (2), which consists of an electrically conducting material and is connected with and makes contact with a plug (3) for connection to a neuro-stimulator. The plug (3) for electrical contact between the active pole of the neuro-stimulator and the stylet (2) has a body (31) extending into a coupling stump (32) and two contact pins (33, 34), the first contact pin (33) being rigidly attached to the body (31) and the second contact pin (34) being aligned with the axis of the coupling stump (32) and being resiliently attached to the body (31).

fig.1

Instrument for local anaesthesia and accompanying plug.

The invention relates to an instrument for local anaesthesia comprising a tubular canula and a stylet including a sharply pointed shaft fitting in the canula.

Furthermore, the invention relates to a plug for electrical contact between the active pole of a neuro-stimulator and a stylet mounted in a coupling socket and inserted into a canula.

The success of conducting anaesthesia wherein, for anaesthetization of a certain tissue or body part, a local anaestheticum is injected in or around the attending nerve branch(es), is strongly dependent on the possibility of depositing the anaestheticum as close as possible to the nerve fibres.

The object of the invention is to provide an instrument which permits a canula to be introduced so that its end will be as close as possible to the nerves to be anaesthetized and at every desired moment a quantity of anaestheticum can be deposited through the canula directly at the location of the nerve to be treated. A subsequent purpose of the invention is to provide a plug permitting in a simple fashion an electrical contact to be made between a known and commercially available catheter including a canula and a stylet, on the other hand, and the active pole of a neuro-stimulator emitting an electrical pulse stimulating the nerves, on the other hand.

Accordingly, the invention provides an instrument as described in the preamble, characterized in that the canula consists of an electrically insulating material and in the operative position of the instrument surrounds the shaft of the stylet, and the stylet consists of an electrically conducting material, and that the stylet is connected with and makes contact with a plug for connection to a neuro-stimulator.

Furthermore, the invention provides a plug, described in the preamble, characterized by a body extending into a coupling stump and by two contact pins, the

0158397

first contact pin being rigidly attached to the body and the second contact pin being aligned with the axis of the coupling stump and being resiliently attached to the body.

The instrument of the invention is particularly suited for introducing a canula to a nerve branch, for example the plexus brachialis for carrying out a plexus block for anaethetizing the arm. In the operative position of the instrument of the invention the shaft of the metal stylet is surrounded by the electrically insulating canula and the stimulating pulse generated by the neuro-stimulator is transmitted by the plug to the free point of the stylet. When a passage for the canula to the nerve plexus is provided by means of the stylet, a nerve excitation synschronous with the electrical pulse and made visible by muscle movements, will be produced by the electrical pulse of suitable frequency, generated on the point of the stylet as soon the point of the stylet has reached the interior of the neuro-vasculator sheath, or lies in the direct vicinity of the nerve branch. Thus, an accurate localization of the point of the stylet and therewith, of the end of the canula is made possible.

The plug has the advantage of being detachable from the stylet.

Preferably, the stylet consists of a hollow puncturing needle so that occasionally it can be observed when a blood vein has been punctured inadvertently, in which case blood will issue through the hollow puncturing needle. The plug of the present invention offers a particularly favourable possibility of converting, by a simple intervention, a commercially available catheter including a canula and a stylet into an instrument according to the invention.

The plug of the invention thus functions as an adapter for connecting an already existent catheter to an already existing neuro-stimulator.

Preferably, the second contact pin of the plug is resiliently mounted within the first contact pin and both contact pins are aligned with the axis of the plug body extending into a coupling stump, in such manner that when the plug is positioned on a coupling socket disposed at the outer end of the stylet the second pin is resiliently

0158397

pressed against the end of the stylet.

However, when the stylet has the shape of a hollow puncturing needle it is desirable, as already stated, that blood can flow outwardly through the puncturing needle. In this case it should be avoided that the second contact pin closes the puncturing needle, and for this reason the second contact pin preferably is provided with an enlarged head which is provided with at least one discharge channel, for example a toothed crown. In such case, when the second contact pin bears against the puncturing needle, the blood will drain between the teeth of the crown.

Whereas in the plug embodiment of the invention as a described adapter the first contact pin provides a connector point for the active pole of a neuro-stimulator, it will be clear that this first contact pin itself also may constitute this active pole and may be permanently connected to the neuro-stimulator.

The invention will now be further explained by way of the attached drawing.

Fig. 1 of the drawing is a longitudinal section of an embodiment of the instrument of the invention.

Fig. 2 is a view of the embodiment of fig. 1 with the three components taken apart.

Fig. 3 is a section of a preferred embodiment of the plug of the invention.

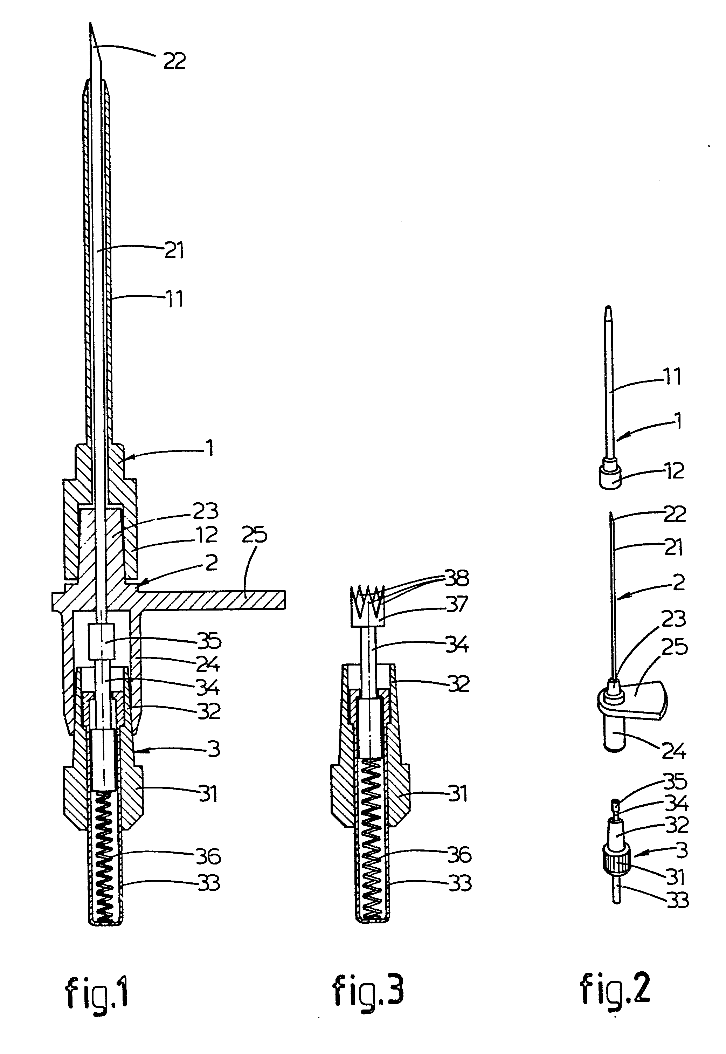

The embodiment of the instrument of the invention illustrated in fig. 1 and 2 comprises a canula 1, a stylet 2 and a plug 3. The canula 1 consists of a sleeve 11 fabricated from an electrically insulating plastic, for example Venflon [R]. At the one end of the sleeve 11 is attached a coupling socket 12 having an inner surface in the shape of a truncated cone of standardized dimensions. In the coupling socket 12 fits the still to be described coupling stump of the stylet 2, as well as the standardized coupling stump of a syringe whereby the anaestheticum is injected after the canula 1 has been brought into the right position.

The stylet 2 comprises a shaft 21 extending into a sharp point 22 whereby the skin of the patient may be easily punctured. The shaft 21 can be a solid rod of a conductive material, in particular metal, but in the illustrated embodiment the shaft 21 is constituted by a hollow needle so that a blood vessel having been punctured will be indicated by the outflowing blood. The shaft 21 is mounted in a coupling stump 23 in the shape of a truncated cone fitting in the coupling socket 12 of the canula 1. The diameter and length of the sleeve 11 and the shaft 21 are mutually adapted so that in the operative position of the instrument with the coupling stump 23 fitted in the coupling socket 12 the sleeve closely surrounds the shaft 21 and completely envelops it except for the free point 22.

The coupling stump 23 of the stylet 2 joins into a coupling socket 24 having preferably the same standardized inner surface as the coupling socket 12 of the canula 1. A grip 25 provides for a good handling capability of the instrument. The coupling stump 23, the coupling socket 24 and the grip 25 are integrally made and usually fabricated of a plastic. Thus the grip is insulated from the electrical circuit.

The plug 3 includes a body 31 fabricated of plastic and extending into a coupling stump 32 fitting in the coupling socket 24. In the body 31 a first contact pin 33, for example of copper, is rigidly attached. The first contact pin 33 is aligned with the axis of the coupling stump 32 and the portion projecting from the body constitutes a connector point for the pole of a neuro-stimulator. In the hollow, first contact pin 33 protrudes a second contact pin 34, for example also of copper, which is outwardly pressed by a spring 36 lying in the first contact pin 33. An abutment edge of the first contact pin 33 and an enlargement of the second contact pin 34 prevent the ejection of the second contact pin 34. By pressure on the free end, in the illustrated case an enlarged head 35, of the second contact pin 34 this can be pushed against the spring more or less into the first contact pin 33. In the operative position of the instrument the plug 3 with the coupling stump 32 is inserted in the fitting coupling socket 24 of the stylet and the second contact pin 34 is pushed against the end 26 of the shaft 21 by the spring 36. The enlarged

**0158397**

head 35 of the second contact pin 34 ensures sufficient surface contact between the second contact pin 34 and the shaft 21.

Fig. 3 shows a preferred embodiment of a plug according to the invention, which as described above comprises a body 31, a coupling stump 32, two contact pins 33, 34 and a spring 36. However, since, as already stated, in case of a puncturing needle used as a shaft 21 it is not desired that the head 35 of the second contact pin completely closes the exit 26 of the puncturing needle, the head of the second contact pin 34 in this preferred embodiment is formed as a crown 37 which is provided with teeth 38. When this preferred plug is positioned on the stylet, the end 26 of the puncturing needle or shaft 21 is engaged by the slanting teeth 38 of the crown 37, the dimensions of the teeth 38 being such that a passage remains free from the interior of the puncturing needle 21 to the interior of the mount 23, 24, 25 of the stylet 2, which usually consists of transparant material, so that issuing blood is visible.

**0158397**

C L A I M S.

1. Instrument for local anaesthesia comprising a tubular canula and a stylet including a sharply pointed shaft fitting in the canula, characterized in that the canula consists of an electrically insulating material and in the operative position of the instrument surrounds the shaft of the stylet, and the stylet consists of an electrically conducting material, and that the stylet is connected with and makes contact with a plug for connection to a neuro-stimulator.

2. Instrument of claim 1, characterized in that the connector plug is being detacheble from the stylet.

3. Instrument of claim 1 or 2, characterized in that the stylet consists of a hollow puncturing needle.

4. Plug for electrical contact between the active pole of a neuro-stimulator and a stylet mounted in a coupling socket and inserted into a canula, characterized by a body (31) extending into a coupling stump (32) and by two contact pins (33, 34), the first contact pin (33) being rigidly attached to the body (31) and the second contact pin (34) being aligned with the axis of the coupling stump (32) and being resiliently attached to the body.

5. Plug of claim 4, characterized in that the two contact pins (33, 34) are both aligned with the axis of the coupling stump (32), and one end of the second contact pin (34) is received into the first contact pin (33) and abuts a spring (36) lying within the first contact pin (33).

6. Plug of claim 4 or 5, characterized in that the free end of the second contact pin is provided with an enlarged head (37) including at least one discharge channel.

fig.1          fig.3          fig.2